(19) Europäisches Patentamt
European Patent Office
Office européen des brevets

(11) EP 0 355 908 B1

(12) EUROPEAN PATENT SPECIFICATION

(45) Date of publication and mention
of the grant of the patent:
**18.12.1996 Bulletin 1996/51**

(51) Int Cl.6: **C08L 5/12**, C12N 1/00,
B01J 13/00, A61K 7/48

(21) Application number: **89202066.0**

(22) Date of filing: **10.08.1989**

(54) **Liquid based composition comprising gelling polysaccharide capable of forming a reversible gel and a method for preparing such composition**

Flüssiges Gemisch, das ein zur Bildung eines reversiblen Gels befähigtes Polysaccharid-Verdickungsmittel enthält und Verfahren zu seiner Herstellung

Composition à base d'un liquide comprenant un polysaccharide gélifiable pouvant former und gel réversible et méthode pour préparer une telle composition

(84) Designated Contracting States:
**AT BE CH DE ES FR GB GR IT LI NL SE**

(30) Priority: **17.08.1988 EP 88201754**

(43) Date of publication of application:
**28.02.1990 Bulletin 1990/09**

(73) Proprietors:
• **UNILEVER N.V.**
  **3013 AL Rotterdam (NL)**
  Designated Contracting States:
  **BE CH DE ES FR GR IT LI NL SE AT**
• **UNILEVER PLC**
  **London EC4P 4BQ (GB)**
  Designated Contracting States:
  **GB**

(72) Inventors:
• **Brown, Charles Rupert Telford**
  **Bedford, MK40 3PS (GB)**
• **Cutler, Alan Norman**
  **Northampton, NN3 2QY (GB)**
• **Norton, Ian Timothy**
  **Rushden Northants, NN10 9EZ (GB)**

(74) Representative: **Kan, Jacob Hendrik, Dr. et al**
**Unilever N.V.**
**Patent Division**
**P.O. Box 137**
**3130 AC Vlaardingen (NL)**

(56) References cited:
EP-A- 0 237 120        EP-A- 0 271 132
EP-A- 0 398 412        DE-A- 2 608 387
FR-A- 2 328 739        US-A- 4 242 239

• **MANUFACTURING CHEMIST AND AEROSOL NEWS, vol. 39, no. 4, April 1968, page 23, left-hand column, Morgan-Grampiann Ltd, London, GB; "Satiagum standard"**

## Description

The present invention is concerned with a liquid-based, composition comprising at least one gelling polysaccharide capable of forming a reversible gel and a method of preparing such a liquid based composition. By a reversible gel is meant a gel that melts when heated and again forms a gel when cooled down subsequently. Examples of gelling polysaccharides capable of forming reversible gels are: agar, carrageenan, furcelleran, gellan and pectin.

The preparation of reversible gels has extensively been described in the prior art. One very well-known example is the preparation of agar gels, used to form agar plates which are a common biological growth support. Agar plates are conventionally made by pouring a heated agar solution into Petri dishes and leaving the dishes undisturbed while the solution gels to an immobile state.

A specific form of reversible gels is found in edible dispersions of water-in-oil where the aqueous phase is present in the form of gelled droplets. E.g.

EP-A-0 237 120 discloses the preparation of fat-continuous spreads which mostly contain gelatin (a protein) as gelling agent which requires at least hours to gel. Ex. 7 discloses the preparation of a fat-continuous dispersion containing 80 wt.% aqueous phase with carrageenan in the form of droplets. The aqueous phase was prepared, pasteurized and cooled to 60°C, the fatty phase was heated to 60°C and 4 parts aqueous phase and 1 part fatty phase were continuously fed into a series of two micro Votator C-units, the first one being operated at 2.000 rpm and the second one at 1,000 rpm. The product left the first unit with a temperature of about 20°C and the second one at about 17°C. The residence time in each equipment unit where shearing took place was less than one minute and there are no indications that during a substantial part of the period the aqueous phase gelled to almost completion the mixture was being sheared. Evidently after phase inversion a fat continuous product was being sheared transferring mechanical energy only to the fatty phase. EP-A-0 271 132 discloses both water-in-oil dispersions and oil-in-water emulsions. Detailed Example 1 is clear in stating on page 20 that a water-continuous emulsion was prepared cooled and so sheared as to prevent setting of the iota carrageenan and then inverted to a fat continuous product. Example 3 is similar here also in the first step an oil-in-water emulsion was prepared. This emulsion (not the aqueous phase!) was pasteurized at 80°C and put through a C-unit at 1000 rpm and a scraped surface heat exchanger at 1000 rpm wherein the fat partially crystallized, gelation took place later in a cooling coil (see also claim 21) and there has been no formation of gelled aqueous beads at this stage but of small oil/fat particles dispersed in an aqueous matrix. As far as this document discloses oil-in-water edible dispersions these do not comprise oil droplets and aqueous microgel particles containing the gelling agent.

In a first aspect the present invention provides a spreadable liquid-based, water-continuous composition comprising a liquid based composition in the form of a microgel having a number weighted mean equivalent diameter of less than 100 micrometer, said microgel containing 0.1 to 15 wt% of at least one gelling polysaccharide in an amount capable of quiescent gelation to form a reversible gel, wherein the liquid-based composition is fluid at a temperature T which is at least 10°C below the gel-setting temperature and wherein G' (shear modulus) is at least 5 times as low as the G' of the gel obtained after heating and subsequent cooling of the composition under quiescent conditions to the same temperature T, and wherein the shear modulus is determined by a Bohlin VOR Rheometer or equivalent apparatus with a 30 mm plate and a 5 degrees cone geometry and water bath, wherein the sample is allowed 10 minutes to equilibrate and the frequency of oscillation is 1Hz and the strain 7.2 degrees.

According to another aspect the present invention provides such a composition, wherein the gelling polysaccharide (s) are selected from the group consisting of agar, kappa-carrageenan, iota-carrageenan, gellan, furcelleran and mixture thereof.

According to another aspect the present invention provides such a composition, wherein the composition comprises at least 50 wt% of water.

According to another aspect the present invention provides such a composition contains from 5 to 30 wt% fat and, in combination, at least 90 wt% of water and fat.

According to a further aspect the present invention provides a method of preparing a spreadable liquid-based water-continuous composition as described above wherein water containing a gelling polysaccharide is subjected to sufficient shear while cooling from a temperature of at least 10°C above the gel-setting temperature to a temperature of at least 10°C below the gel-setting temperature and wherein the shearing is started before G' (shear modulus) assumes 5% of its equilibrium value, and shearing is not stopped before the G' assumes 90% of its equilibrium value to obtain a spreadable, water-continuous liquid-based composition wherein the shear modulus is determined by a Bohlin VOR Rheometer or equivalent apparatus with a 30 mm plate and a 5 degrees cone geometry and water bath, wherein the sample is allowed 10 minutes to equilibrate and the frequency of oscillation is 1Hz and the strain 7.2 degrees.

The temperature T preferably is within the range of 0°C to 100°C, more preferably in the range of 5°C to 50°C.

The transition temperature of a particular composition is the temperature at which, upon slow temperature increase, the ordered form, be it of microscopical or macroscopical size, has disappeared completely. The transition temperature can be measured by means of differential scanning calorimetry. The transition temperature of the present composition can be somewhat higher than its gel melting point.

The gel melting point of the present composition can be determined by measuring the melting point of a gel, containing exactly the same ingredients, obtained by cooling under quiescent conditions. By cooling under quiescent conditions is to be understood cooling in the absence of any agitation. Here the term agitation encompasses actions such as shearing, stirring and shaking.

The melting temperature of a gel can suitably be measured using the following procedure: Pour a sample into a glass test tube and allow it to set fully at 5°C. Then place the tube in a water jacket connected to a programmable water bath. Place a steel ball, having a diameter of approximately 1 mm, on the surface of the sample and depress slightly in order to minimize surface tension effects. Equilibrate for one hour at 25°C, or a lower temperature in case of a low melting gel, and then apply a heating regime of 0.05°C/min. The gel melting point is the temperature at which the ball begins to sink through the sample. Movement of the ball can be observed using a travelling microscope.

The fluid composition according to the present invention offers several advantages. The present composition may be stored indefinitely in its more mobile state, but can be returned to its normal more rigid state at any moment, by simply heating through the transition temperature of said composition, whereafter the heated solution will form a normal gel on cooling under quiescent conditions.

Due to its mobile state, the present fluid composition equals a pourable fluid, capable of flow and of forming a level surface. The mobile state of the fluid composition according to the invention thus enables easy transportation. The composition is easily pumpable and can be converted to a stable rigid gel whenever desired. The rigid gel thus obtained is moreover indistinguishable from a gel originally prepared from the same ingredients by heating and subsequent cooling under quiescent conditions.

The present fluid composition furthermore possesses the advantageous property that it disrupts at low strain (e. g. rubbing or mastication) to give a very smooth, fat-like consistency. This property makes that the fluid composition can suitably be turned into products such as skin creams, moisturizer lotions, spreads, etc., by the inclusion of suitable ingredients (e.g. perfume, colouring, flavouring). The smooth disruption at low strain of the present composition is essentially different from the fracturing observed when rigid gels are subjected to conditions of strain.

The fluid composition according to the invention can advantageously be used as a culture medium in e.g. Petri dishes. A proportioned amount of the fluid composition can simply be poured into a Petri dish and act as a culture medium. If a rigid growth support is desired, a measured amount of the fluid composition can be poured onto a Petri dish, after which the Petri dish is heated to above the gel melting point and left to cool. Thus a rigid culture medium is formed which is indistinguishable from a medium obtained via conventional methods well known in the art.

Another advantage of the present fluid composition is that it can suitably be used in the preparation of spreads. The fluid composition according to the invention, comprising more or less discrete gelled particles, can advantageously be dispersed in a fat matrix so as to obtain a stable water-in-oil dispersion. In this manner dispersions can be prepared that contain water-soluble ingredients which would hinder the preparation of a water-in-oil dispersion when using conventional processing.

The use of the fluid composition according to the invention in the preparation of water-in-oil emulsions such as spreads furthermore offers the advantage that the size of the gelled droplets in the emulsion depends heavily on the size of the gelled beads in the fluid composition. Indeed, when the fluid composition according to the invention is incorporated into a continuous fat phase, using conventional emulsification techniques, the mean equivalent diameter of the microgels before and after emulsification are not substantially different. Thus the droplet size can be controlled more easily than in a conventional process in which the (heated) gelling agent containing aqueous phase is emulsified into the fat phase.

The mobile condition of the present composition should not be confused with the enhancement of mobility which can be achieved by breaking up a gel into particles which can roll and slide over one another. The gel particles thus obtained are of macroscopical size and are visible to the naked eye, i.e. they have an average size exceeding 100 microns. Thus effectively such a broken gel is composed of numerous relatively large fragments, possessing the same properties, e.g. rigidity, as the original gel. Another noticeable difference between broken gels and the compositions according to the invention is that broken gels display a clear tendency to loose liquid.

As contrasted with broken gels, the present fluid composition, when viewed under a microscope, is composed of very small microgels, which microgels have a mean equivalent diameter not exceeding 100 microns, more preferably not exceeding 50 microns. Here the mean equivalent diameter is the number weighted mean equivalent diameter of the microgels having an equivalent diameter in the range of 0.1 to 200 microns. Most preferably the microgels present in the liquid based composition of the invention have a mean equivalent diameter in the range of 0.1 to 30 microns.

The equivalent diameter distribution of the microgels can suitably be established from microscopical images. Although such may be done by hand, in order to obtain more reproducible results, it is preferred to determine the diameter distribution by means of an image analyzing computer. Of course the magnification applied should be suitable for properly determining the diameter distribution. For determining the diameter distribution in the range of 0.1 to 100 microns a magnification of 800 appeared to be appropriate. Furthermore it should be observed that the mean equivalent diameter found, when using an image analyzing computer, may depend on the particle concentration, since overlapping particles may not be recognized as separate particles, but instead be regarded as one larger particle.

Although the polysaccharide microgels in the present composition can have a size of less than 0.1 micron or more than 200 microns, preferably, more than 90%, more preferably more than 95% by weight of the polysaccharides is present in the form of microgels having an equivalent diameter in the range of 0.1 to 200 microns, more preferably in the range of 0.1 to 100 microns.

The low rigidity of the present composition in comparison with a gel obtained by quiescently cooling an identical composition through its gel setting point, can be illustrated by comparing the shear modulus of both compositions, at the same temperature. The shear modulus of the present fluid composition, at a temperature T, preferably is at least 5 times as low as the shear modulus of the gel obtained after the heating and subsequent cooling under quiescent conditions to the same temperature T.

The shear modulus of a composition can be determined by using the method described further on in this application, be it that, in order to establish the shear modulus at a certain temperature T, the sample is equilibrated at that temperature without precedent heating to above the transition temperature.

By the gel setting point as referred to in this application is meant the temperature at which, upon slowly cooling down, an ordered structure if formed. The gel setting point for a composition according to the invention can be determined by heating the composition to above the transition point, splitting it up in a number of samples which are subsequently equilibrated, under quiescent conditions, at different temperatures lying 1 centigrade apart, and dropping a steel ball of about 1 mm diameter on each of the samples after equilibration. If the samples are ordered in accordance with the temperature at which the samples were equilibrated, starting from the sample equilibrated at the highest temperature, the gel setting temperature is the equilibration temperature of the first sample through which the steel ball did not sink.

In an even more preferred embodiment, the shear modulus of the fluid composition according to the invention, at a temperature T, is less than 200 Pa., more preferably the shear modulus is within the range of 1 to 150 Pa., and the shear modulus of the gel obtained after heating and subsequent quiescent cooling of the composition to the same temperature T, is at least 100 Pa., more preferably at least 200 Pa.

The present invention is applicable to gelling polysaccharides whose solutions naturally form gels comprising a macroscopical three dimensional network structure. A number of polysaccharides display this characteristic in a very pronounced manner, in particular agar, kappa-carrageenan, iota-carrageenan and furcelleran.

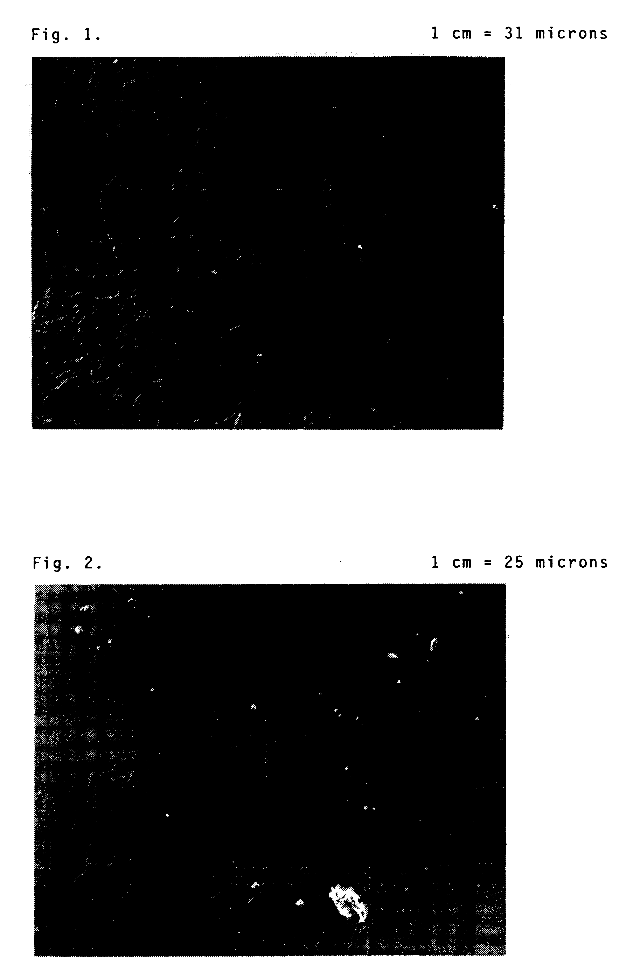

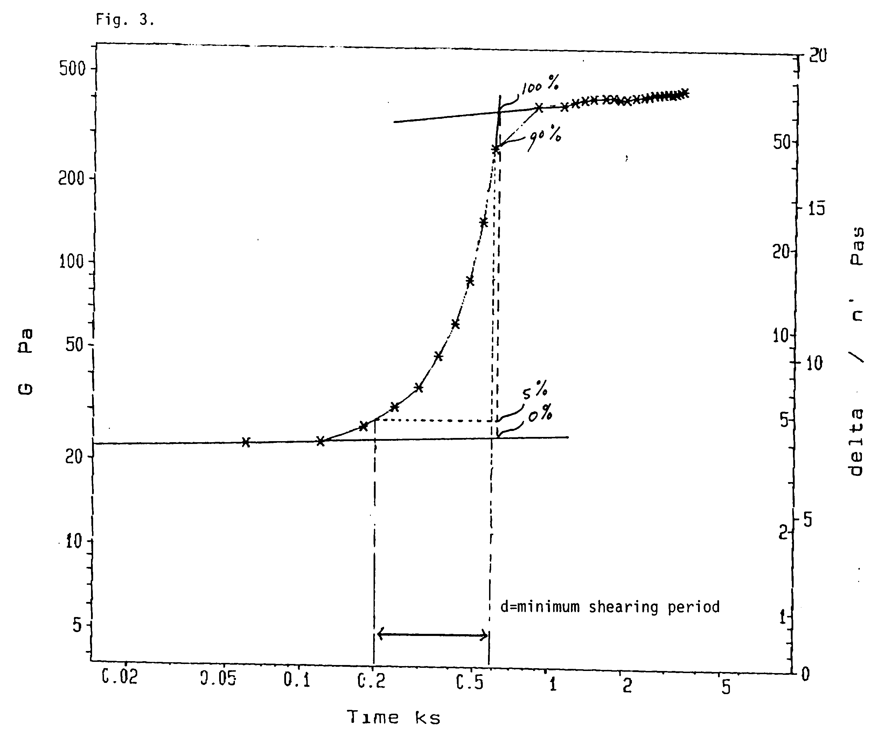

Although we do not wish to be bound by theory it is believed that the mobile state of the present fluid composition may be explained from the absence, on a macroscopical scale, of a three dimensional network. As can be seen from figures 1 and 2, respectively representing a photograph of the microscopical image of a pourable composition according to the present invention and a micro-photograph of the same composition diluted with a factor 10, said pourable composition is composed of essentially non-aggregated microgels. As can be derived from figures 1 and 2, the microgels in the present composition may be of a spherical or irregular shape.

The present fluid composition is preferably based on a polar liquid. More preferably said liquid is selected from the group consisting of: water, ethanol, isopropanol and mixtures thereof. For most purposes water is the most suitable liquid to be used. Preferably the present composition, at room temperature, comprises at least 50 wt.%, more preferably at least 70 wt.% of the liquid.

The liquid based composition according to the present invention, besides liquid and gelling polysaccharides, can also comprise other components. Such additional components may be immiscible with the liquid in which the gelling polysaccharide is dissolved. Thus the present composition can contain two or more distinct phases. Although, in case the additional immiscible components account for the major part of the composition, it may be argued that such a product is not a liquid based composition, such a composition is nevertheless encompassed by the present application. Indeed the presence of a relatively mobile, gelling polysaccharide containing liquid, is also appreciated in such products containing at least two distinct phases. An example of a fluid composition according to the present invention containing a component which is immiscible with the liquid, is a composition comprising a continuous aqueous phase containing the gelling polysaccharide and a fat phase which is dispersed in said aqueous phase.

The fluid composition according to the present invention, due to its special rheology and provided the right ingredients are added thereto, can advantageously be utilized as a product for personal care including cosmetic products, such as skin cream, moisturizer lotion, hair gel, deodorant, anti-perspirant etc., or as a pharmaceutical product such as ointment. The present fluid composition usually forms the bulk of such pharmaceutical products or products of personal care.

The present composition can beneficially be employed in both relatively viscous systems such as ointments and skin creams and less viscous systems such as deodorants. When utilized in ointments and skin creams the present composition offers the advantage that an appropriate rheology can be obtained without the need of using fatty emulsions which give a persisting greasy skin feel.

EP 0 355 908 B1

When used in, for instance, deodorants, the present composition offers the advantage that a viscous fluid composition is obtained by the inclusion of natural polymers, rather than the synthetic polymers normally used. The known inclusion of gelling polysaccharides in deodorants has the disadvantage that a more or less rigid gel is formed, hampering discharge from the container the deodorant is held in.

Examples of substances that may suitably be included in the pharmaceutical products and products for personal care according to the present invention are:

(i) Perfumes, which give rise to an olfactory response in the form of a fragrance, and deodorant perfumes, such as those described in US patent 4,278,658 (Lever Brothers & Co) which in addition to providing a fragrance response, can also reduce body malodour;

(ii) Skin coolants, such as menthol, menthyl lactate, menthyl pyrrolidone carboxylate N-ethyl-p-menthane-3-carboxamide and other derivatives of menthol, which give rise to a tactile response in the form of a cooling sensation on the skin; and

(iii) Emollients such as isopropylmyristate, silicone oils, mineral oils and vegetable oils which give rise to a tactile response in the form of an increase in skin lubricity.

(iv) Deodorants other than perfumes, whose function is to reduce the level of or eliminate microflora at the skin surface, especially those responsible for the development of body malodour. Precursors of deodorants other than perfume can also be employed.

(v) Antiperspirant actives, whose function is to reduce or eliminate the appearance of perspiration at the skin surface.

(vi) Anticholinergic actives, whose function is to reduce or eliminate the generation of perspiration before it reaches the skin surface, examples of which are scopolamine derivatives, such as scopolamine hydrobromide and esters thereof, such as benzoyl scopolamine hydrobromide.

(vii) Emulsifiers
Emulsifiers may be nonionic, anionic or cationic.
Examples of suitable emulsifiers are:

|  | HLB value |
| --- | --- |
| BRIJ 72 (Polyoxyethylene-2-stearyl ether) | 4.9 |
| BRIJ 52 (Polyoxyethylene-2-cetyl ether) | 5.3 |

(viii) Activity Enhancer
The composition according to the invention can also optionally comprise an activity enhancer, which can be chosen from a wide variety of molecules. Particular classes of activity enhancers include penetration enhancers and cationic polymers. Reference is made to co-pending European patent application No. 89304841.3 in which various suitable activity enhancers are mentioned.

(ix) Suncreens
The emulsion according to the invention can also optionally comprise one or more sunscreen active ingredients.

The amount of the sunscreen active ingredient that can optionally be employed in accordance with the invention, as a therapeutically effective amount, will normally be from 0.01 to 10%, preferably from 0.1 to 5% and most preferably from 1 to 5% by weight of the composition.

In addition to the above ingredients conventionally used in products for personal care, the composition according to the invention can optionally comprise ingredients such as a colourant, preservative, antioxidant, in amounts which are conventional in the cosmetics, pharmaceutical etc.

The present fluid gelling polysaccharide containing composition, when having an appropriate rheology and containing particular ingredients such as flavouring, colouring, preservative, fat, etc., can suitably constitute a food product such as a spread, in particular a low calorie spread.

The composition according to the invention can contain various other ingredients such as colouring agent, flavour-

5

ing, fat, salt, anti-oxidant, emulsifier etc. Preferably the composition according to the invention the composition is edible and contains, in combination, at least 90 wt.% of water and/or fat. Most preferably the present composition is water-continuous and contains from 5 to 30 wt.% fat.

The liquid in the present fluid composition preferably contains gelling polysaccharide(s) in an amount capable of quiescent gelation. Here by the liquid is meant that liquid phase in the product in which the gelling polysaccharide dissolves most easily. The amount of gelling polysaccharide can lie below 12 wt.%, but even higher concentrations are possible. A more preferred range of gelling polysaccharide, calculated on the liquid, is 0.1-15 wt.%. For agar a preferred range is 0.2 5% by weight of the liquid. For carrageenans and furcelleran a preferred range is 0.5 to 10% by weight of the liquid. According to a preferred embodiment of the invention the liquid constitutes a continuous phase in the present composition.

The composition according to the invention can contain materials which have the effect of modifying the gel melting and setting temperature, for example ion-sources such as salts, which have a strong influence on the gel melting and setting point.

Another aspect of the present invention is a method of preparing a fluid composition containing a sufficient amount of at least one gelling polysaccharide to form a reversible gel on quiescent cooling, wherein a liquid containing the gelling polysaccharide(s) is subjected to sufficient shear while cooling the liquid through its gel setting temperature to obtain a substantially less rigid composition than would be obtained by quiescent cooling.

Although it is possible to obtain the advantages of the present process whilst cooling down through a very narrow temperature range, preferably shear is applied while cooling down the liquid containing the gelling polysaccharide(s) through a temperature range of at least 10°C. More preferably shear is applied while cooling down the liquid containing the gelling polysaccharide(s) from at least 10°C above the gel setting point to at least 10°C below the gel setting point.

The following method can be used to determine, for a given composition and at a certain cooling rate, the shearing time required to obtain a suitable product: Fit a Bohlin VOR Rheometer, or equivalent apparatus, with a 30 mm plate and 5 degree cone geometry and water bath. Set the water bath temperature at least 10°C higher than the transition temperature of the material to be tested. Place the liquid sample of the polysaccharide to be tested between the cone and the plate and set the gap at the plate edge to be 1 mm. Apply silicone oil to the plate edge to prevent the sample from drying out.

Allow ten minutes for the sample to equilibrate with the water bath temperature. Meanwhile, set the frequency of oscillation (f) to be 1 Hz and the strain to be 7.2 degrees. Set a rate of cooling equal to or at least similar to that used in the method of preparing the fluid composition and cool to a temperature of at least 10°C below the gel setting temperature of the test material.

Start continuous sinusoidal oscillation and record compliant strain at convenient time intervals (t) during cooling and for at least 1 hour after the lower temperature has been reached.

Calculate the shear storage modulus (G') from the relationship:

$$G' = (\eta_{21}°/\gamma_{21}°) \cos \delta$$

where

$$\eta_{21}° = \eta_{21} / \sin (\omega t + \delta)$$

$$\gamma_{21}° = \gamma_{21} / \sin \omega t$$

$\eta$ is the shear stress
$\gamma$ is the shear strain
and $\delta$ is the phase angle

More detailed information can be found in "Viscoelastic Properties of Polymers" by J.D. Ferry, Chapter 1, pages 4-16, Std Book Number 471 25774 5, published by J. Wiley & Sons Inc.

The equilibrium value of G', which can be derived from a plot of G' versus time, is then characteristic of the gel formed. An example is given in figure 3 for 50:50 kappa/iota carrageenan (1% w/w) in aqueous NaCl (1.5% w/w) and potassium sorbate (0.2% w/w) solution.

Referring to the above method of analysis, in the present method of preparing a fluid composition, the shearing is preferably started before G' assumes 5%, more preferably 2% of its equilibrium value, and shearing is not stopped before the G' assumes 90%, more preferably 95% of its equilibrium value. In order to get a useful indication as to the period during which shear must be applied in the present process, it is crucial that the cooling regimes applied in the method of analysis is very similar to the cooling regime applied in said process.

The present process can be carried out as a batch process, but it is preferred to carry out the process in a continuous or semi-continuous manner by, for instance, continuously passing a (pre-heated) stream of gelling polysaccharide containing liquid through one or more cooling and shearing units.

The present invention encompasses a process for preparing a fat-continuous dispersion, containing a dispersed aqueous phase, by inversion of an oil-in-water emulsion to a water-in-oil dispersion. In the latter process an oil-in-water emulsion containing a sufficient amount of gelling polysaccharide to gel the aqueous phase, is cooled through the gel setting temperature under conditions of shear. Thus the continuous aqueous phase is converted to a fluid composition composed of microgels. After the formation of the microgels the cooling and shearing conditions will induce the inversion of the water-continuous emulsion to a water-in-oil dispersion.

The present invention also encompasses the application of more than one gelling polysaccharide. In case the present gelling polysaccharides containing liquid comprises two or more gelling polysaccharides at respective concentration levels exceeding the critical concentration of the polysaccharide in said composition, the liquid must be sheared through the gel setting points of each of said polysaccharides. The critical concentration of a gelling polysaccharide in a particular composition can be calculated from measurements of the shear modulus of a series of samples containing different concentrations of gelling agent, as described in Br. Polymer J. 17 (1985), 164.

The invention is further illustrated by means of the following examples:

## Example 1

A formulation was prepared consisting of 1 wt.% agar (Oxoid$^{TM}$ L11) and 99 wt.% deionised water. The agar was dissolved in the water at 80°C after which the solution was warmed to 95°C and then allowed to cool slowly in a glass beaker while stirring vigorously with a magnetic stirrer. Cooling was continued until the stirred solution reached a temperature of 13°C. For cooling below room temperature the solution the beaker was immersed in ice water.

The solution was allowed to warm to 20°C. Both at 13°C and 20°C the solution was a pourable liquid. It was found that this liquid was stable and could be stored at room temperature for six months without apparent change in its condition. Other materials could additionally be stirred into the liquid, again without change in its condition.

A sample of the liquid was reheated to 95°C and then allowed to cool without agitation. It cooled to a rigid gel which was indistinguishable from the gel obtained by cooling, under quiescent conditions, a sample of the original heated solution of agar. The shear modulus of the rigid gel obtained after reheating is more than 5 times as high as the shear modulus of the pourable liquid obtained through sheared cooling.

## Example 2

Example 1 was repeated but after dissolving the agar and warming the solution to 95°C it was cooled in a continuous process with throughput of 25g per minute. The solution was cooled during passage through four units of apparatus. In the first, third and fourth units (known as Votators$^{R}$) the solution was subjected to shear by scraping over a cooled surface. In the second unit (called a C-unit) the solution passed through the annular space between a rotor bearing radially projecting pins and a surrounding cooled jacket from which pins projected radially inwardly towards the rotor. The jackets of all four pieces of apparatus were cooled by water at 10°C.

The agar solution obtained by cooling with this continuous process was again a stable pourable liquid which could be stored and which could be converted to a rigid gel by heating to 95°C and then allowing to cool without disturbance. The shear modulus of the rigid agar gel obtained after reheating the pourable liquid is more than 5 times as high as the shear modulus of the pourable liquid obtained through sheared cooling.

## Example 3

Example 2 was repeated using a formulation consisting of 2% agar and 98% deionised water and cooling under shear in a single C-unit to a pourable liquid which could be converted to a self supporting rigid gel by reheating to 95°C and allowing to cool undisturbed. The shear modulus of the rigid gel obtained after reheating is more than 5 times as high as the shear modulus of the pourable liquid obtained through sheared cooling.

The pourable liquid was viewed under a microscope at a magnification of x320. A photograph of the image obtained is represented in figure 1. The pourable liquid was subsequently diluted by a factor 10 with deionised water after which a sample was taken and viewed under a microscope at magnification x400. A photograph of the image is represented in figure 2.

The mean equivalent diameter of the microgels present in the sample obtained after dilution was determined by means of a Quantimet 970 particle size analyzer. The mean equivalent diameter, having measured 514 microgels, was found to be about 10 microns. Less than 5% of the particles appeared to have an equivalent diameter of more than 25 microns. In fact the true mean equivalent diameter was even less than 10 microns as some overlap between microgels was observed.

Example 4

A formulation was used consisting of:

1.3% carrageenan
0.41% sodium chloride
1.05% potassium sorbate
-- balance deionised water --

The carrageenan was kappa and iota carrageenan in a weight ratio of 70:30.

The carrageenan and the salts were dissolved in the deionised water at 80°C and briefly heated to 95°C. The solution was allowed to cool to 66°C and then cooled from 66°C to 30°C while applying shear. Cooling while applying shear was carried out by passing the solution through a cooled Votator[R].

The cooled composition was a thick pumpable liquid which, when heated slowly, showed a rapid loss of viscosity at approximately 55°C. The shear modulus of the rigid gel obtained after reheating to 95°C and subsequent cooling is more than 5 times as high as the shear modulus, measured at the same temperature, of the pourable liquid obtained through sheared cooling.

Example 5

A formulation was prepared containing:
1.3% carrageenan (70:30 kappa:iota)
0.41% sodium chloride
0.15% potassium sorbate
-- balance deionised water --

The solids were dissolved in the water at 80°C and then cooled while shearing as described in example 4. After cooling the product was a thick liquid which, upon slowly heating, displayed rapid thinning at 35°C.

Example 6

Examples 4 and 5 were both repeated using a carrageenan which was a blend of equal parts of kappa and iota carrageenan. Very similar results were obtained but it was found necessary to apply more vigorous shear to the solution during cooling.

Example 7

A formulation was prepared consisting of:
4% carrageenan (kappa:iota 85:15)
0.41% sodium chloride
1.05% potassium sorbate
-- balance deionised water --

The solids were dissolved in deionised water at 80°C and then cooled while applying shear as in example 4. The product after cooling was liquid with a rather thick consistency.

Example 8

A formulation was prepared consisting of:
4% kappa carrageenan
9% hydrolysed starch
0.41% sodium chlorid
1% potassium sorbate
-- balance deionised water --

The procedure of Example 4 was again followed. The product after cooling with shear had a consistency resembling petroleum jelly. Aggregation of the hydrolysed starch in the product had increased the light scattering of the solution so that the product had an opaque appearance.

Example 9

Example 4 was repeated using 1% carrageenan which was a blend of 60:40 kappa:iota carrageenans. Essentially similar results were obtained.

Example 10

A moisturizer lotion was made on the basis of the following ingredients:

| Ingredient | wt.% |
|---|---|
| Kappa-carrageenan X6960 [1] | 1.05 |
| Iota-carrageenan X6955 [1] | 0.45 |
| Glycerol | 3.00 |
| Sodium chloride | 0.60 |
| Nipagen™ M [2] | 0.10 |
| Colouring agent | 0.03-0.06 |
| Flavouring | 0.10 |
| Water | balance |

[1] ex Copenhagen Pectin Factory, Copenhagen, Denmark

[2] Sodium methyl para benzoate, preservative, ex NIPA Laboratories, LLantwit Fadre, Pontypridd, Wales

Four different samples were made in the same manner as described in Example 4, using different colouring agents and flavouring, namely:

| Sample | Colouring Agent | Flavour |
|---|---|---|
| (a) Pink | 0.05% Natural Pink | Raspberry oil |
| (b) Blue | 0.06% Indigo Carmine | Cherry oil |
| (c) Yellow | 0.06% Crocin | Eucalyptus Citriodora oil |
| (d) Green | 0.03 Copper Chlorophyll | Apple oil |

The lotions obtained when rubbed over the skin, appeared to be both cating and moisturizing. After a short period of rubbing the skin appeared to be very smooth and non-greasy.

Example 11

A moisturizer cream was made on the basis of the following ingredients:

| Ingredient | wt.% |
|---|---|
| Kappa-carrageenan X6960 | 0.840 |
| Iota-carrageenan X6955 | 0.360 |
| Glycerol BP | 3.000 |
| Sodium chloride | 0.410 |
| Potassium chloride | 0.045 |
| Colouring agent | 0.03-0.06 |
| Flavouring | 0.10 |
| Water | balance |

Four different samples were made in the same manner as described in Example 4, using different colouring agents and flavouring, namely:

| Sample | Colouring Agent | Flavour |
|---|---|---|
| (a) Pink | 0.05% Natural Pink | Raspberry oil |
| (b) Blue | 0.06% Indigo Carmine | Cherry oil |
| (c) Yellow | 0.06% Crocin | Eucalyptus Citriodora oil |
| (d) Green | 0.03 Copper Chlorophyll | Apple oil |

The moisturizer creams were found to have a clear smoothening and moistering effect when rubbed over the skin.

Example 12

A cleanser gel was prepared in the same manner and from the same ingredients as in Example 11, with the exception that instead of glycerol, the starting material contained 7.5 wt.% Brij 58 (Nonionic surfactant ex. Sigma Chemicals Ltd., Poole, Dorset BH17 7NH, England).

Example 13

A water-continuous vaseline-like product was prepared from the following ingredients:

| Ingredient | wt.% |
|---|---|
| Kappa-carrageenan X6960 | 4.00 |
| Sodium chloride | 0.41 |
| Potassium sorbate | 1.05 |
| Glycerol | 3.00 |
| Water | balance |

The above composition was processed by passing the hot mixture held at 93°C, through a scraped surface heat exchanger (Votator$^{TM}$) operating as follows:

| Unit speed | 4200 rpm |
|---|---|
| Jacket temperature | 16°C |
| Inlet temperature | 68°C |
| Exit temperature | 38°C |
| Feed rate | 50 g/min |

The product so obtained appeared to have a gel melting point of about 55°C and had a viscosity and skin feel very similar to vaseline.

Example 14

A water-continuous spread was prepared on the basis of the following formulation:

| Ingredient | wt.% |
|---|---|
| Kappa-Carrageenan X6960 | 2 |
| Palm kernel oil (hardened to a slip melting point of 38°C) | 24 |
| Sunflower oil | 16 |
| Sodium chloride | 0.5 |
| Potassium sorbate | 1.05 |
| Sodium caseinate | 0.6 |
| Flavour & beta carotene | |
| Water | to 100 |

The formulation was selected to give a gel melting temperature of 49°C. The formulation was made up by dissolving the water soluble ingredients to which the melted fat was added and then homogenised at 2000 psi using a Crepeco™ homogeniser. The pH was then adjusted to 5.8 using lactic acid.

The mixture was then processed by passing it through a cooled scraped surface heat exchanger (Votator™) running at 3000 rpm, with the jacket temperature set to decrease the temperature from 90 to 30°C at a throughput of 100g/min.

A second product was made using a fat phase of 60 wt.% hycoa 5, 40 wt.% sunflower oil and an aqueous phase of 3.3 wt.% kappa carrageenan, 1.75% potassium sorbate and 0.85 wt.% sodium chloride. This gave a gel melting temperature of 67°C. Both products were smooth and spreadable with no oiling off even when the temperature was cycled between 45°C and 35°C for 1 month. Thus products of this type are very suited for production and sale in tropical countries.

Example 15 (Comparative)

A fat-continuous spread was prepared from the following formulation:

| Aqueous phase | |
|---|---|
| Sodium chloride | 1.0 wt.% |
| Potassium sorbate | 0.08 wt.% |
| Agar | 1.2 wt.% |
| Water | 57.72 wt.% |
| pH adjusted 5.5 with lactic acid | |
| Fat phase | |
| Hardstock | 17.6 wt.% |
| Sunflower oil | 22 wt.% |
| Monoglycerides | 0.2 wt.% |
| Lecithin | 0.2 wt.% |
| Colouring and flavouring | |

The aqueous phase, having an initial temperature of 90°C, was sheared and cooled in two cooled C-units, both running at 1400 rpm and with a final exit temperature of approximately 20°C. The microgels in the product so obtained had a volume weighted mean diameter of 10 microns and was stored at 4°C overnight. After storage the aqueous phase was heated to 40°C and mixed with the fat phase. A spread was made using a sequence consisting of a scraped surface heat exchanger, a C-unit, a second scraped surface heat exchanger and a second C-unit. The throughput employed was 50 g/min with shaft speeds of approximately 1000 rpm. The dispersed aqueous phase was found to have a volume weighted mean diameter of 9 microns.

Similar results were obtained when the agar concentration in the aqueous phase was reduced from 1.2 wt.% to 0.6 wt.%, with no holding overnight.

Example 16

A growth support medium was prepared from the following formulation:

| Ingredient | wt.% |
|---|---|
| Oxoid™ L11 Agar | 1 |
| Oxoid™ Broth No 2 | 2.5 |
| Water | to 100 |

The ingredients were dissolved into the water at 90°C. A sequence of four cooled shearing devices (a scraped surface heat exchanger (A-unit) followed by a C-unit, a second scraped surface heat exchangers and a second C-unit) were washed with ethanol and then agar and broth were passed through. The processing conditions employed were as follows:

| Processing unit | rpm | exit temperature |
|---|---|---|
| A-unit 1 | 4000 | 23°C |
| C-unit | 4000 | 24°C |
| A-unit 2 | 1500 | 21°C |
| C-unit 3 | 1500 | 12°C |

A smooth pourable liquid was obtained at a throughput of 50g/min, which pourable liquid did not form a rigid gel on holding at 5°C for weeks.

**Claims**

1. Spreadable liquid-based, water-continuous composition comprising a liquid based composition in the form of a microgel having a number weighted mean equivalent diameter of less than 100 micrometer, said microgel containing 0.1 to 15 wt% of at least one gelling polysaccharide in an amount capable of quiescent gelation to form a reversible gel, wherein the liquid-based composition is fluid at a temperature T which is at least 10°C below the gel-setting temperature and wherein G' (shear modulus) is at least 5 times as low as the G' of the gel obtained after heating and subsequent cooling of the composition under quiescent conditions to the same temperature T, and wherein the shear modulus is determined by a Bohlin VOR Rheometer or equivalent apparatus with a 30 mm plate and a 5 degrees cone geometry and water bath, wherein the sample is allowed 10 minutes to equilibrate and the frequency of oscillation is 1Hz and the strain 7.2 degrees.

2. A composition according to claim 1, wherein the gelling polysaccharide(s) are selected from the group consisting of agar, kappa-carrageenan, iota-carrageenan, gellan, furcelleran and mixtures thereof.

3. A composition according to any one of claims 1-2, wherein the composition comprises at least 50 wt% of water.

4. A composition according to any one of claims 1-3, wherein the composition contains from 5 to 30 wt% fat and, in combination, at least 90 wt% of water and fat.

5. Method of preparing a spreadable liquid-based, water-continuous composition according to claim 1; wherein water containing a gelling polysaccharide is subjected to sufficient shear while cooling from a temperature of at least 10°C above the gel-setting temperature to a temperature of at least 10°C below the gel-setting temperature and wherein the shearing is started before G'(shear modulus) assumes 5% of its equilibrium value, and shearing is not stopped before the G' assumes 90% of its equilibrium value to obtain a spreadable, water-continuous liquid-based composition wherein the shear modulus is determined by a Bohlin VOR Rheometer or equivalent apparatus with a 30 mm plate and a 5 degrees cone geometry and water bath, wherein the sample is allowed 10 minutes to equilibrate and the frequency of oscillation is 1Hz and the strain 7.2 degrees.

**Patentansprüche**

1. Streichfähiges, wasserkontinuierliches, flüssiges Gemisch bzw. Zusammensetzung auf Flüssigkeitsbasis, das/die ein Gemisch auf Flüssigkeitsbasis in Form eines Mikrogels mit einem zahlengewichteten mittleren Äquivalentdurchmesser von weniger als 100 Mikrometern umfaßt, wobei das Mikrogel 0,1 bis 15 Gew.-% mindestens eines gelierenden Polysaccharids in einer Menge enthält, die zu einer ruhenden Gelierung unter Bildung eines reversiblen Gels befähigt ist, worin das flüssige Gemisch bei einer Temperatur T fluid ist, die mindestens 10°C unterhalb der Gelerstarrungstemperatur liegt, und worin G' (Schermodul) mindestens fünfmal niedriger als der G'-Wert des Gels ist, das nach Erhitzen und anschließendem Abkühlen des Gemischs unter ruhenden Bedingungen auf dieselbe Temperatur T erhalten worden ist, und worin der Schermodul durch ein Bohlin VOR-Rheometer oder eine äquivalente Vorrichtung mit einer 30 mm-Platte und einer Konusgeometrie von 5° und einem Wasserbad bestimmt wird, worin man die Probe 10 Minuten äquilibrieren läßt und in der die Frequenz der Oszillation 1 Hz und die Deformation 7,2° betragen.

2. Gemisch nach Anspruch 1, worin das oder die gelierenden Polysaccharide aus der aus Agar, kappa-Carrageenan,

**EP 0 355 908 B1**

iota-Carrageenan, Gellan, Furcelleran und Mischungen davon bestehenden Gruppe ausgewählt ist.

3. Gemisch nach irgendeinem der Ansprüche 1 bis 2, worin das Gemisch mindestens 50 Gew.-% Wasser umfaßt.

4. Gemisch nach irgendeinem der Ansprüche 1 bis 3, worin das Gemisch 5 bis 30 Gew.-% Fett und, in Kombination, mindestens 90 Gew.-% Fett und Wasser enthält.

5. Verfahren zur Herstellung eines streichfähigen, wasserkontinuierlichen, flüssigen Gemischs nach Anspruch 1, bei dem das ein gelierendes Polysaccharid enthaltende Wasser einer ausreichenden Scherung unterworfen wird, während man es von einer Temperatur mindestens 10°C oberhalb der Gelerstarrungstemperatur auf eine Temperatur mindestens 10°C unterhalb der Gelerstarrungstemperatur abkühlt, und worin die Scherung gestartet wird, bevor G' (Schermodul) 5 % seines Gleichgewichtswertes annimmt, und die Scherung nicht gestoppt wird, bevor G' 90 % seines Gleichgewichtswertes annimmt, um ein streichfähiges, wasserkontinuierliches, flüssiges Gemisch zu erhalten, wobei der Schermodul durch ein Bohlin VOR-Rheometer oder eine äquivalente Vorrichtung mit einer 30 mm-Platte und einer Konusgeometrie von 5° und einem Wasserbad bestimmt wird, worin man die Probe 10 Minuten äquilibrieren läßt und in der die Frequenz der Oszillation 1 Hz und die Deformation 7,2° betragen.

**Revendications**

1. Composition qu'on peut tartiner continue en eau, à base d'un liquide comprenant une composition à base d'un liquide sous forme d'un microgel ayant un diamètre équivalent moyen pondéré en nombre inférieur à 100 micromètres, ledit microgel contenant 0,1 à 15% en poids d'au moins un polysaccharide gélifiant en une quantité capable de gélification au repos pour former un gel réversible, la composition à base de liquide étant fluide à une température T qui est d'au moins 10°C en dessous la température de prise du gel et G' (module de cisaillement) étant au moins 5 fois aussi bas que G' du gel obtenu après chauffage et ensuite refroidissement de la composition dans des conditions de repos à la même température T, le module de cisaillement étant déterminé par un rhéomètre BOHLIN VOR ou un appareil équivalent avec une plaquette de 30 mm et un cône à 5° et un bain d'eau dans lequel les échantillons sont laissés 10 minutes pour s'équilibrer, la fréquence d'oscillation étant de 1 Hz et la contrainte de 7,2°.

2. Composition selon la revendication 1, dans laquelle le (ou les) polysaccharide gélifiant est choisi parmi l'agar agar, le kappa-carragheenane, le iota-carragheenane, le gellane, le furcellerane et leurs mélanges.

3. Composition selon l'une quelconque des revendications 1 à 2, dans laquelle la composition comprend au moins 50% en poids d'eau.

4. Composition selon l'une quelconque des revendications 1 à 3, dans laquelle la composition contient de 5 à 30% en poids de graisse, et en combinaison, au moins 90% en poids d'eau et de graisse.

5. Procédé de préparation d'une composition continue en eau, à base de liquide qu'on peut tartiner selon la revendication 1, dans lequel l'eau contenant un polysaccharide gélifiant est soumise à un cisaillement suffisant tout en refroidissant à partir d'une température d'au moins 10°C au dessus de la température de prise de gel jusqu'à une température d'au moins 10°C en dessous de la température de prise de gel, le cisaillement débutant avant que G' (module de cisaillement) n'atteigne 5% de son degré d'équilibre, et le cisaillement n'étant pas arrêté avant que G' n'atteigne 90% de son degré d'équilibre pour obtenir une composition qu'on peut tartiner, continue en eau, à base d'un liquide, le module de cisaillement étant déterminé par un rhéomètre BOHLIN VOR ou un appareil équivalent ayant une plaquette de 30 mm et un cône à 5° et un bain d'eau dans lequel les échantillons sont laissés 10 minutes pour s'équilibrer, la fréquence d'oscillation étant de 1 Hz et la contrainte de 7,2°.

13

Fig. 1.                    1 cm = 31 microns

Fig. 2.                    1 cm = 25 microns

Fig. 3.